# EUROPEAN PATENT APPLICATION

(11) **EP 4 806 500 A1**
(43) Date of publication of application: **16.09.2026**
(21) Application number: 26161364.0
(22) Date of filing: 27.02.2026
(51) Int. Cl.: A61N 1/36, A61B 5/389, A61B 5/296

(54) **MUSCLE RELAXATION MONITORING DEVICE AND MEDICAL DEVICE SYSTEM**

(30) Priority: 14.03.2025 JP 2025041821
(71) Applicant: Nihon Kohden Corporation, Shinjuku-ku Tokyo (JP)
(72) Inventor: Yoshihara, Hiroshi, Tokorozawa-shi, Saitama (JP)
(74) Representative: Grünecker Patent- und Rechtsanwälte PartG mbB

(57) **Abstract**

A muscle relaxation monitoring device includes: a stimulator configured to stimulate a nerve of a living body via a stimulation electrode attached to the living body; a physiological signal detector configured to detect a physiological signal via a recording electrode attached to the living body; and a detachment detector configured to detect detachment of the recording electrode based on a physiological waveform corresponding to the physiological signal detected by the physiological signal detector.

## Description

### TECHNICAL FIELD

The presently disclosed subject matter relates to a muscle relaxation monitoring device and a medical device system.

### BACKGROUND ART

In the related art, when performing surgery, an anesthetic is administered for the purpose of safe surgery. However, even if a patient loses consciousness or feeling due to the administration of the anesthetic, a muscle of the patient may contract due to reflection or the like. When the muscle of the patient contracts, there is a possibility that the surgery cannot be performed safely, and therefore, when performing large surgery, a muscle relaxant is administered to the patient. After the muscle relaxant is administered, a muscle relaxation degree is monitored by a muscle relaxation monitoring device.

Monitoring of the muscle relaxation degree by the muscle relaxation monitoring device is generally performed by a train of four (TOF) method in which muscle stimulation is repeated four times every 0.5 sec and the muscle stimulation is repeated four times every 15 sec (for example, JP2006-326050A). In a case where the muscle relaxation degree is monitored by the TOF method, the muscle relaxation degree is generally monitored using two stimulation electrodes and two recording electrodes.

In recent years, there has been known an invention for specifying an attachment position between a stimulation electrode and a recording electrode and detachment of a neutral electrode (JP2024-167794A). Specifically, a nerve of the patient is stimulated via the stimulation electrode, and a process of specifying the attachment position of the stimulation electrode or the recording electrode or the detachment of the neutral electrode is performed based on a physiological signal detected via the recording electrode. Accordingly, since it is possible to specify the attachment position of the stimulation electrode or the recording electrode or the detachment of the neutral electrode, it is possible to quickly respond to the attachment position of the stimulation electrode or the recording electrode or the detachment of the neutral electrode, and to quickly perform preparation before performing surgery.

In the case where the muscle relaxation degree is monitored by the TOF method disclosed in JP2006-326050A, it is necessary to attach a recording electrode to a patient. In order to detect whether the recording electrode is attached to the patient, an impedance check function is required.

However, in order to provide the impedance check function, there is a problem that a complicated circuit configuration such as providing an input/output circuit in the recording electrode is required. In addition, when the input/output circuit is provided in the recording electrode, there is a problem in that a circuit scale increases, which leads to an increase in cost. On the other hand, a muscle relaxation monitoring device disclosed in JP2024-167794A can detect detachment of the neutral electrode, but cannot detect whether the recording electrode is attached.

### SUMMARY

In view of the above problems, it is an object of the presently disclosed subject matter to provide a muscle relaxation monitoring device capable of detecting whether a recording electrode is attached to a patient without requiring a complicated circuit configuration.

In order to solve such a problem, a muscle relaxation monitoring device according to the presently disclosed subject matter includes a stimulator configured to stimulate a nerve of a living body via a stimulation electrode attached to the living body, a physiological signal detector configured to detect a physiological signal via a recording electrode attached to the living body, and a detachment detector configured to detect detachment of the recording electrode based on a physiological waveform corresponding to the physiological signal detected by the physiological signal detector.

According to the presently disclosed subject matter, it is possible to provide a muscle relaxation monitoring device capable of detecting whether a recording electrode is attached to a patient without requiring a complicated circuit configuration.

### BRIEF DESCRIPTION OF DRAWINGS

FIG. 1 illustrates a configuration of a medical device system according to a first embodiment.
FIG. 2 illustrates an electromyogram waveform in a case where a recording electrode is attached.
FIG. 3 illustrates an electromyogram waveform in a case where the recording electrode is not attached.
FIG. 4 is a flowchart of a process performed by a muscle relaxation monitoring device.
FIGS. 5A to 5C are timing charts of the muscle relaxation monitoring device.
FIG. 6 illustrates a configuration of a medical device system according to a second embodiment.

### DESCRIPTION OF EMBODIMENTS

### First Embodiment

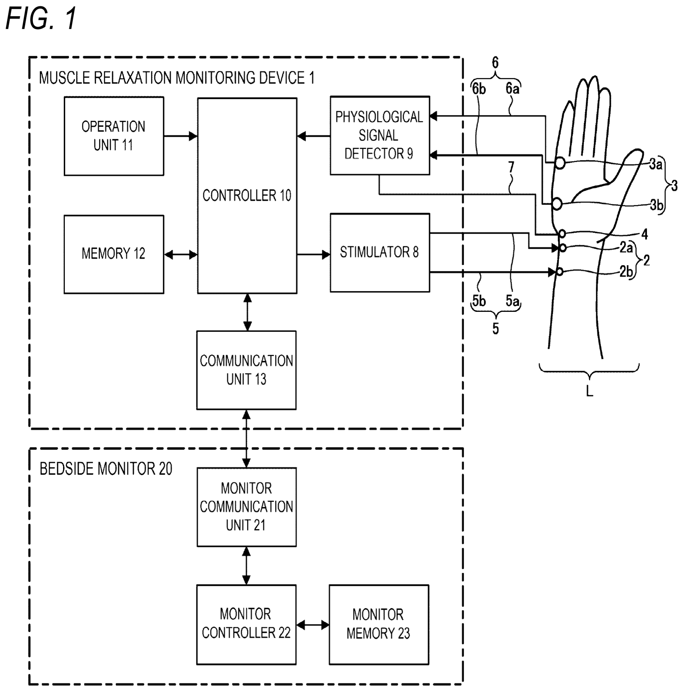

Hereinafter, a first embodiment of the presently disclosed subject matter will be described with reference to FIGS. 1 to 5C.

### Medical Device System in First Embodiment

First, a medical device system according to the first embodiment of the presently disclosed subject matter will be described with reference to FIG. 1.

The medical device system according to the first embodiment can include a muscle relaxation monitoring device 1, a stimulation electrode 2, recording electrodes 3, a neutral electrode 4, a wiring 5, a signal line 6, a ground line 7, and a bedside monitor 20.

The muscle relaxation monitoring device 1 is a device for monitoring a muscle relaxation degree of a patient who is a living body to which a muscle relaxant is administered. The muscle relaxation monitoring device 1 can include a stimulator 8, a physiological signal detector 9, a controller 10, an operation unit 11, a memory 12, and a communication unit 13.

The stimulation electrode 2 can include a first stimulation electrode 2a and a second stimulation electrode 2b, and is attached to a position corresponding to an ulnar nerve of an upper limb L of the patient. The stimulation electrode 2 is connected to the stimulator 8 via the wiring 5. Specifically, the first stimulation electrode 2a is connected to the stimulator 8 via a first wiring 5a. On the other hand, the second stimulation electrode 2b is connected to the stimulator 8 via a second wiring 5b.

The recording electrode 3 can include a first recording electrode 3a and a second recording electrode 3b, and is attached to positions corresponding to the abductor digiti minimi muscle of the upper limb L of the patient and a tendon in the vicinity thereof. The recording electrode 3 is connected to the physiological signal detector 9 via the signal line 6. Specifically, the first recording electrode 3a is connected to the physiological signal detector 9 via a first signal line 6a. On the other hand, the second recording electrode 3b is connected to the physiological signal detector 9 via a second signal line 6b.

The neutral electrode 4 is provided to reduce an influence of a noise voltage transmitted from the body of the patient through the upper limb L on a physiological signal detected through the recording electrode 3. The neutral electrode 4 is connected to the physiological signal detector 9 via the ground line 7. The noise voltage is caused by propagation of a voltage pulse caused by the stimulation current in the body of the patient.

The wiring 5 can include the first wiring 5a and the second wiring 5b, and is provided to connect the stimulation electrode 2 and the stimulator 8. Specifically, the first wiring 5a is provided to connect the first stimulation electrode 2a and the stimulator 8. On the other hand, the second wiring 5b is provided to connect the second stimulation electrode 2b and the stimulator 8.

The signal line 6 can include the first signal line 6a and the second signal line 6b, and is provided to connect the recording electrode 3 and the physiological signal detector 9. Specifically, the first signal line 6a is provided to connect the first recording electrode 3a and the physiological signal detector 9. On the other hand, the second signal line 6b is provided to connect the second recording electrode 3b and the physiological signal detector 9.

The ground line 7 is provided to connect the neutral electrode 4 and the physiological signal detector 9. The stimulator 8 is provided to stimulate the ulnar nerve of the upper limb L of the patient by applying a pulsed stimulation current through the wiring 5 and the stimulation electrode 2. The physiological signal detector 9 detects a potential difference between two physiological signals generated in the recording electrode 3 and the abductor digiti minimi muscle and the tendon of the upper limb L of the patient input via the signal line 6. The stimulator 8 constitutes a "stimulator" in the presently disclosed subject matter, and the physiological signal detector 9 constitutes a "physiological signal detector" in the presently disclosed subject matter.

The controller 10 can include a CPU and the like, and controls the stimulator 8 and the communication unit 13 by reading an operation program stored in the memory 12 and performing a predetermined calculation process. In addition, the controller 10 performs a process of storing information on the physiological signal input from the physiological signal detector 9 in the memory 12. In addition, the controller 10 controls the muscle relaxation monitoring device 1 when a signal output from the operation unit 11 is input.

The operation unit 11 is provided to input a command from a user of the muscle relaxation monitoring device 1. The operation unit 11 may be a button, a touch panel, a keyboard, a mouse, a trackball, or the like.

The memory 12 is provided to store an operation program of the muscle relaxation monitoring device 1, a result of the predetermined calculation process performed by the controller 10, and the like. The memory 12 may be a recording medium such as a hard disk, a flexible disk, an MO, an MT, a RAM, a CD-ROM, a DVD-ROM, an SD card, a CF card, or a USB memory, or a server.

The communication unit 13 is provided to output a predetermined calculation result by the controller 10 to a monitor communication unit 21. Specifically, the communication unit 13 is provided to output a monitoring result of the muscle relaxation degree by the muscle relaxation monitoring device 1 and information indicating that the recording electrode 3 is not attached (hereinafter, referred to as "detachment information") to the monitor communication unit 21. The communication unit 13 is provided to input predetermined information from the monitor communication unit 21.

The "detachment information" is information on detachment of the recording electrode 3, and can include information indicating that the first recording electrode 3a is attached and the second recording electrode 3b is detached, information indicating that the second recording electrode 3b is attached and the first recording electrode 3a is detached, and information indicating that both the first recording electrode 3a and the second recording electrode 3b are detached.

The bedside monitor 20 is an example of a medical device, and is provided to display a predetermined calculation result output from the communication unit 13. Specifically, the bedside monitor 20 is provided to display the monitoring result of the muscle relaxation degree by the muscle relaxation monitoring device 1 and the detachment information. The bedside monitor 20 can include the monitor communication unit 21, a monitor controller 22, and a monitor memory 23.

The monitor communication unit 21 is provided to input the predetermined calculation result output from the communication unit 13. The monitor communication unit 21 is provided to output a calculation result by the monitor controller 22 to the communication unit 13.

The monitor controller 22 can include a CPU and the like, and controls the bedside monitor 20 by reading an operation program stored in the monitor memory 23 and performing a predetermined calculation process. The monitor memory 23 is provided to store the operation program of the bedside monitor 20, a result of the predetermined calculation process performed by the monitor controller 22, and the like. The monitor memory 23 may be a recording medium such as a hard disk, a flexible disk, an MO, an MT, a RAM, a CD-ROM, a DVD-ROM, an SD card, a CF card, or a USB memory, or a server. The monitor controller 22 constitutes a "medical device controller" in the presently disclosed subject matter.

Here, a method of using the muscle relaxation monitoring device 1 will be described. First, the stimulation electrode 2, the recording electrode 3, and the neutral electrode 4 are attached to the upper limb L of the patient. Next, the stimulator 8 causes a stimulation current to flow between the first stimulation electrode 2a and the second stimulation electrode 2b via the first wiring 5a and the second wiring 5b to stimulate the ulnar nerve of the upper limb L of the patient. Next, the physiological signal detector 9 detects the potential difference between the two physiological signals generated in the abductor digiti minimi muscle and the tendon of the upper limb L of the patient based on the flow of the stimulation current by the stimulator 8. Then, based on the potential difference of the physiological signal detected by the physiological signal detector 9, a physiological waveform (electromyogram waveform) is obtained to monitor the muscle relaxation degree of the patient.

### Electromyogram Waveform in Case Where Recording Electrode 3 is Attached

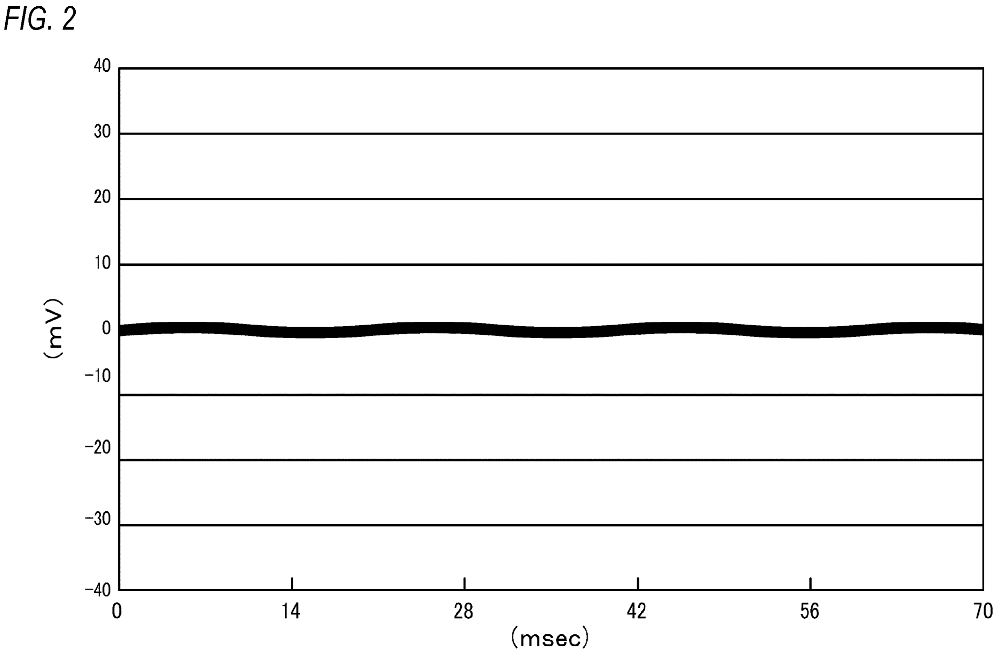

Next, an electromyogram waveform in a case where the recording electrode 3 is attached will be described with reference to FIG. 2.

In FIG. 2, a vertical axis represents a potential difference (unit: mV), and a horizontal axis represents time (unit: msec). The electromyogram waveform is obtained by detecting the potential difference of the physiological signal from the two recording electrodes 3 attached to the upper limb L of the patient and by the potential difference of the detected physiological signal. When the physiological signal is input to the physiological signal detector 9 via the recording electrode 3, an AC component is superimposed on the physiological signal. Here, in a case where the two recording electrodes 3 are attached to the upper limb L of the patient and the stimulation current is not flowing by the stimulator 8, since the AC component is minute, the electromyogram waveform transitions around 0 mV, which is the base line.

### Electromyogram Waveform in Case Where Recording Electrode 3 is not Attached

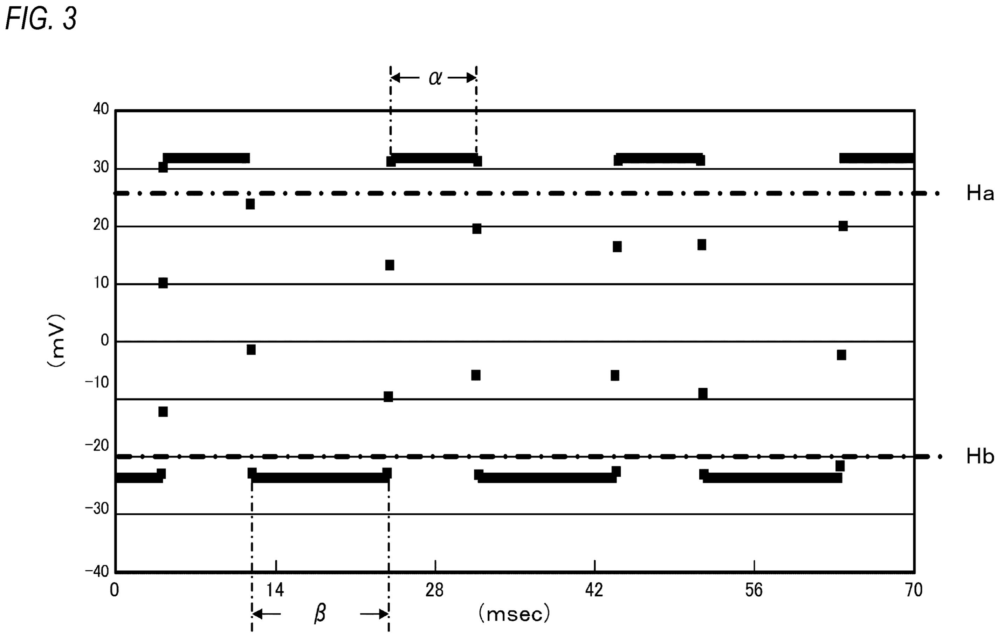

Next, an electromyogram waveform in a case where the recording electrode 3 is not attached will be described with reference to FIG. 3.

In the case where the recording electrode 3 is not attached to the patient, the AC component is greatly amplified, and the electromyogram waveform greatly fluctuates. Specifically, as illustrated in FIG. 3, the electromyogram waveform greatly fluctuates beyond a threshold value, and the electromyogram waveform sticks up and down by the drift of the portion with the large fluctuation. In the case where the recording electrode 3 is not attached to the patient, the electromyogram waveform fluctuates greatly even when the stimulation current is not applied by the stimulator 8.

Here, the "case where the recording electrode 3 is not attached to the patient" is any one of a case where the first recording electrode 3a is attached and the second recording electrode 3b is detached, a case where the second recording electrode 3b is attached and the first recording electrode 3a is detached, and a case where both the first recording electrode 3a and the second recording electrode 3b are detached.

The threshold value is a value that is not normally detected by the potential difference of the physiological signal detected by the physiological signal detector 9, and has a first threshold value Ha and a second threshold value Hb. The first threshold value Ha is a positive value (specifically, "25" mV), and the second threshold value Hb is a negative value (specifically, "-20" mV). The threshold value may be any value as long as it is a value that is not normally detected by the potential difference of the physiological signal detected by the physiological signal detector 9.

Note that α illustrated in FIG. 3 represents a time during which the electromyogram waveform continuously exceeds the first threshold value Ha. Specifically, α is a time longer than a predetermined time (Y msec) to be described later. On the other hand, β illustrated in FIG. 3 represents a time during which the electromyogram waveform continuously exceeds the second threshold value Hb. Specifically, β is a time longer than a predetermined time (Y msec) to be described later.

### Flowchart of Process Performed by Muscle Relaxation Monitoring Device 1

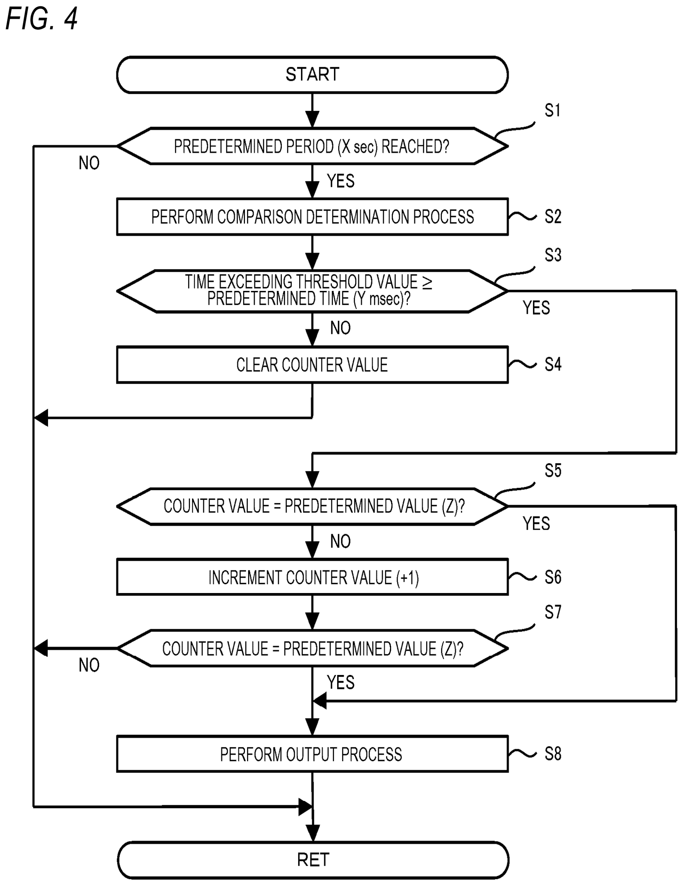

Next, a flowchart of a process performed by the muscle relaxation monitoring device 1 will be described with reference to FIG. 4.

In step S1, the controller 10 performs a process of determining whether a predetermined period (X sec) is reached. When it is determined that the predetermined period (X sec) is reached (step S1 = YES), the process proceeds to step S2. On the other hand, when it is determined that the predetermined period (X sec) is not reached (step S1 = NO), the process performed by the muscle relaxation monitoring device 1 ends. By performing the process of step S1, the processes of step S2 to step S8 performed by the muscle relaxation monitoring device 1 are performed every predetermined period (X sec).

In step S2, the controller 10 performs a comparison determination process of comparing the threshold value described with reference to FIG. 3 with the electromyogram waveform and determining whether the electromyogram waveform continuously exceeds the threshold value for a predetermined time (Y msec). Specifically, the controller 10 first performs a calculation process using the first threshold value Ha and the obtained electromyogram waveform to determine whether the electromyogram waveform has a value larger than the first threshold value Ha continuously for a predetermined time. Next, a calculation process using the second threshold value Hb and the obtained electromyogram waveform is performed to determine whether the value is continuously smaller than the second threshold value Hb for a predetermined time. When the process of step S2 ends, the process proceeds to step S3.

Here, the predetermined time (Y msec) is defined based on a period of an AC frequency. Specifically, a period of a sine wave having an AC frequency of 60 Hz is 1 sec/60 Hz = about 16.6 msec. Since the period of about 16.6 msec is a full wave period, the half wave period is about 8.3 msec, and a value smaller than about 8.3 msec which is the half wave period can be adopted as the predetermined time (Y msec). In other words, the predetermined time (Y msec) may be any time shorter than about 8.3 msec.

In step S3, the controller 10 performs a process of determining whether the value of the electromyogram waveform continuously exceeds the threshold value for a predetermined time. Specifically, as a result of performing the comparison determination process of step S2, the controller 10 performs a process of determining whether the value of the electromyogram waveform continuously exceeds the first threshold value Ha for a predetermined time or whether the value of the electromyogram waveform continuously exceeds the second threshold value Hb for a predetermined time. When it is determined that the value of the electromyogram waveform continuously exceeds the threshold value for the predetermined time (step S3 = YES), the process proceeds to step S5. On the other hand, when it is determined that the value of the electromyogram waveform does not continuously exceed the threshold value for the predetermined time (step S3 = NO), the process proceeds to step S4.

In step S4, the controller 10 performs a process of clearing a counter value. When the process of step S4 ends, the process performed by the muscle relaxation monitoring device 1 ends. By performing the process of step S4, when the value of the electromyogram waveform does not continuously exceed the threshold value for the predetermined time, the counter value is cleared to 0.

In step S5, the controller 10 performs a process of determining whether the counter value is a predetermined value (Z). When it is determined that the counter value is the predetermined value (Z) (step S5 = YES), the process proceeds to step S8. On the other hand, when it is determined that the counter value is not the predetermined value (Z) (step S5 = NO), the process proceeds to step S6. When it is determined that the counter value is the predetermined value (Z) by performing the process of step S5, the process proceeds to step S8, and thus the process of step S6 is not performed. This prevents the counter value from being larger than the predetermined value (Z).

Here, the predetermined value (Z) may be any value, but is preferably a natural number of two or more. For example, as the value of the predetermined value (Z) is set to a larger value, determination accuracy of whether the recording electrode 3 is attached can be improved. On the other hand, as the value of the predetermined value (Z) is set to a smaller value, a determination speed of whether the recording electrode 3 is attached can be improved.

In step S6, the controller 10 performs a process of incrementing the counter value. When the process of step S6 ends, the process proceeds to step S7. By performing the process of step S6, the counter value is incremented by one and updated.

In step S7, the controller 10 performs a process of determining whether the counter value reaches the predetermined value (Z) as a result of incrementing the counter value by the process of step S6. When it is determined that the counter value reaches the predetermined value (Z) (step S7 = YES), the process proceeds to step S8. On the other hand, when it is determined that the counter value does not reach the predetermined value (Z) (step S7 = NO), the process performed by the muscle relaxation monitoring device 1 ends.

In step S8, the controller 10 performs an output process of outputting the detachment information to the monitor communication unit 21 via the communication unit 13. When the process of step S8 ends, the process performed by the muscle relaxation monitoring device 1 ends.

In the present embodiment, the controller 10 that performs the processes of steps S2 to S7 constitutes a "detachment detector" in the presently disclosed subject matter, and the processes of steps S2 to S7 constitute a "detachment determination process" in the presently disclosed subject matter. In the comparison determination process of step S2, the determination that the value of the electromyogram waveform exceeds the threshold value constitutes a "specific result" in the presently disclosed subject matter. In the process of step S6, incrementing the counter value constitutes "counting" in the presently disclosed subject matter, and the process of step S6 constitutes a "counting process" in the presently disclosed subject matter.

### Timing Chart of Muscle Relaxation Monitoring Device

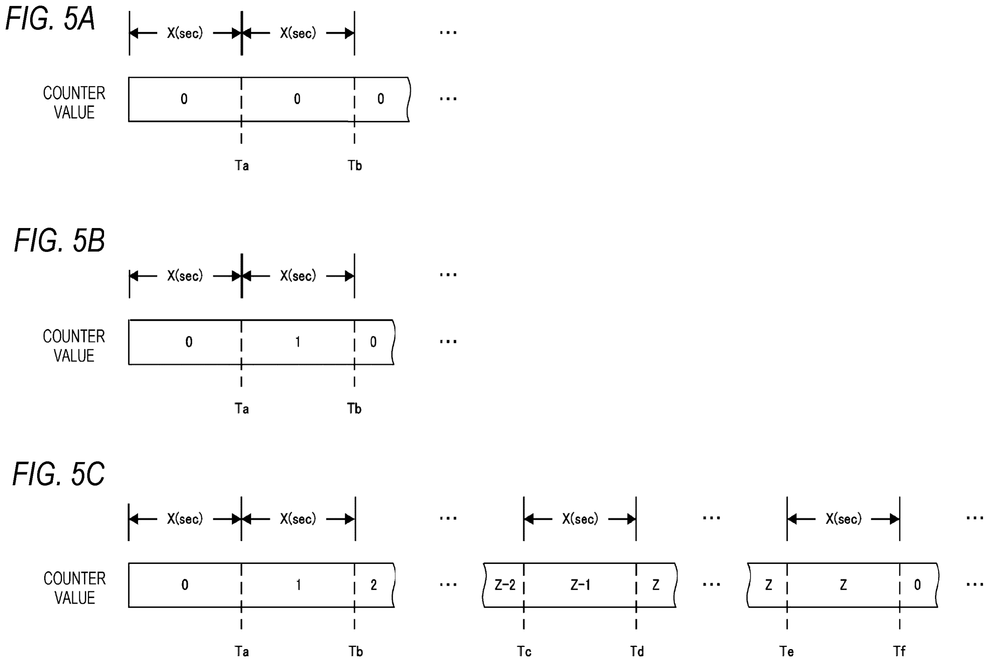

Next, a timing chart of the muscle relaxation monitoring device will be described with reference to FIGS. 5A to 5C.

FIG. 5A is a timing chart in the case where the recording electrode 3 is attached. A timing Ta is a timing at which it is determined that the value of the electromyogram waveform does not continuously exceed the threshold value for the predetermined time (Y msec) in the process performed by the muscle relaxation monitoring device 1 (see FIG. 4) (step S3 = NO). At this time, the counter value is cleared and remains 0 (step S4).

A timing Tb is a timing at which it is determined that the value of the electromyogram waveform does not continuously exceed the threshold value for the predetermined time (Y msec) in the process (see FIG. 4) performed by the muscle relaxation monitoring device 1 to be performed next (step S3 = NO). At this time, the counter value is cleared and remains 0 (step S4).

As described above, when the recording electrode 3 is attached to the upper limb L of the patient, the counter value is cleared and always becomes 0. Therefore, since the counter value does not reach the predetermined value (Z), the detachment information is not output to the monitor communication unit 21.

FIG. 5B is a timing chart in a case where it is determined that the recording electrode 3 is attached before the counter value reaches the predetermined value (Z) after it is determined that the recording electrode 3 is not attached. The timing Ta is a timing at which it is determined that the value of the electromyogram waveform continuously exceeds the threshold value for the predetermined time (Y msec) in the process performed by the muscle relaxation monitoring device 1 (see FIG. 4) (step S3 = YES). At this time, since the counter value is 0 and does not reach the predetermined value (Z) (step S5 = NO), the counter value is incremented (step S6). As a result, the counter value changes from 0 to 1.

A timing Tc is a timing at which it is determined that the value of the electromyogram waveform does not continuously exceed the threshold value for the predetermined time (Y msec) in the process (see FIG. 4) performed by the muscle relaxation monitoring device 1 to be performed next (step S3 = NO). At this time, the counter value is cleared to 0 (step S4). As a result, the counter value changes from 1 to 0.

As described above, even if it is determined that the value of the electromyogram waveform continuously exceeds the threshold value for the predetermined time (Y msec) and the counter value is incremented, when it is determined that the value of the electromyogram waveform does not continuously exceed the threshold value for the predetermined time (Y msec) before the counter value reaches the predetermined value (Z), the counter value is cleared and becomes 0. Therefore, since the counter value does not reach the predetermined value (Z), the detachment information is not output to the monitor communication unit 21.

FIG. 5C is a timing chart in the case where the recording electrode 3 is not attached. The timing Ta is the timing at which it is determined that the value of the electromyogram waveform continuously exceeds the threshold value for the predetermined time (Y msec) in the process performed by the muscle relaxation monitoring device 1 (see FIG. 4) (step S3 = YES). At this time, since the counter value does not reach the predetermined value (Z) (step S5 = NO), the counter value is incremented (step S6). As a result, the counter value changes from 0 to 1.

The timing Tb is the timing at which it is determined that the value of the electromyogram waveform continuously exceeds the threshold value for the predetermined time (Y msec) in the process (see FIG. 4) performed by the muscle relaxation monitoring device 1 to be performed next (step S3 = YES). At this time, since the counter value does not reach the predetermined value (Z) (step S5 = NO), the counter value is incremented (step S6). As a result, the counter value changes from 1 to 2.

The timing Tc is a timing at which it is determined that the value of the electromyogram waveform continuously exceeds the threshold value for the predetermined time (Y msec) in the subsequent process (see FIG. 4) performed by the muscle relaxation monitoring device 1 (step S3 = YES). At this time, since the counter value does not reach the predetermined value (Z) (step S5 = NO), the counter value is incremented (step S6). As a result, the counter value changes from Z-2 to Z-1.

A timing Td is a timing at which it is determined that the value of the electromyogram waveform continuously exceeds the threshold value for the predetermined time (Y msec) in the process (see FIG. 4) performed by the muscle relaxation monitoring device 1 to be performed next (step S3 = YES). At this time, since the counter value does not reach the predetermined value (Z) (step S5 = NO), the counter value is incremented (step S6). As a result, the counter value changes from Z-1 to Z. Then, since the counter value reaches the predetermined value (Z) (step S7 = YES), the detachment information is output to the monitor communication unit 21 (step S8).

A timing Te is a timing at which it is determined that the value of the electromyogram waveform continuously exceeds the threshold value for the predetermined time (Y msec) in the subsequent process (see FIG. 4) performed by the muscle relaxation monitoring device 1 (step S3 = YES). At this time, since the counter value reaches the predetermined value (Z) (step S5 = YES), the detachment information is output to the monitor communication unit 21 (step S8).

A timing Tf is a timing at which it is determined that the value of the electromyogram waveform does not continuously exceed the threshold value for the predetermined time (Y msec) in the process (see FIG. 4) performed by the muscle relaxation monitoring device 1 to be performed next (step S3 = NO). At this time, the counter value is cleared to 0 (step S4). As a result, the counter value changes from Z to 0. Accordingly, the detachment information that has been continuously output to the monitor communication unit 21 is not output.

As described above, when it is determined that the value of the electromyogram waveform continuously exceeds the threshold value for the predetermined time (Y msec) and the counter value reaches the predetermined value (Z), the detachment information is output to the monitor communication unit 21. After the counter value reaches the predetermined value (Z), the detachment information is continuously output to the monitor communication unit 21 until the counter value becomes 0. Thereafter, when it is determined that the electromyogram waveform does not continuously exceed the threshold value for the predetermined time (Y msec), the counter value is cleared to 0, and the detachment information that has been continuously output to the monitor communication unit 21 is not output.

Here, the muscle relaxation monitoring device 1 outputs the detachment information to the monitor communication unit 21 based on the fact that the value of the electromyogram waveform continuously exceeds the threshold value for the predetermined time (Y msec). That is, when the value of the electromyogram waveform simply exceeds the threshold value, the detachment information is not output to the monitor communication unit 21. This is to prevent, for example, an erroneous determination that a noise voltage is mixed due to the use of an electric scalpel and it is determined that the threshold value is exceeded by the noise voltage.

The muscle relaxation monitoring device 1 outputs the detachment information to the monitor communication unit 21 based on the fact that the counter value is incremented when the value of the electromyogram waveform continuously exceeds the threshold value for the predetermined time (Y msec) and the counter value reaches the predetermined value (Z). That is, even if the value of the electromyogram waveform continuously exceeds the threshold value for the predetermined time (Y msec), the detachment information is not output to the monitor communication unit 21. This is also to prevent the erroneous determination that the noise voltage is mixed due to the use of the electric scalpel and it is determined that the threshold value is exceeded by the noise voltage.

### Medical Device System in Second Embodiment

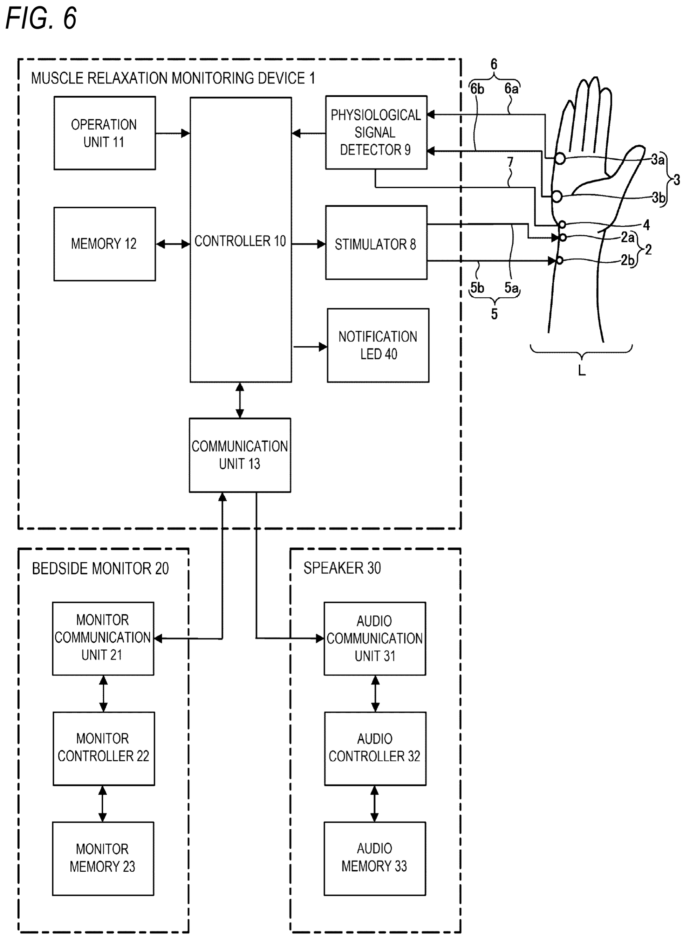

Next, a medical device system according to a second embodiment will be described with reference to FIG. 6.

The medical device system according to the second embodiment can include the muscle relaxation monitoring device 1, the stimulation electrode 2, the recording electrodes 3, the neutral electrode 4, the wiring 5, the signal line 6, the ground line 7, the bedside monitor 20, and a speaker 30.

The muscle relaxation monitoring device 1 according to the second embodiment can include the stimulator 8, the physiological signal detector 9, the controller 10, the operation unit 11, the memory 12, the communication unit 13, and a notification LED 40.

The speaker 30 is provided to notify by audio that the recording electrode 3 is not attached. The speaker 30 can include an audio communication unit 31, an audio controller 32, and an audio memory 33. The audio communication unit 31 is provided to input a predetermined calculation result output from the communication unit 13. The audio controller 32 can include a CPU and the like, and controls the speaker 30 by reading an operation program and audio data stored in the audio memory 33 and performing a predetermined calculation process. The audio memory 33 is provided to store the operation program and the audio data of the speaker 30, a result of the predetermined calculation process performed by the audio controller 32, and the like. The audio memory 33 may be a recording medium such as a hard disk, a flexible disk, an MO, an MT, a RAM, a CD-ROM, a DVD-ROM, an SD card, a CF card, or a USB memory, or a server.

The notification LED 40 is provided to emit light to notify that the recording electrode 3 is not attached. Specifically, the notification LED 40 emits light in a first emission color (for example, green) in the case where the recording electrode 3 is attached to the upper limb L of the patient. On the other hand, the notification LED 40 emits light in a second emission color (for example, red) different from the first emission color in the case where the recording electrode 3 is not attached to the upper limb L of the patient.

Here, the memory 12 in the second embodiment stores light emission data for notifying that the recording electrode 3 is not attached. The controller 10 reads the light emission data stored in the memory 12 and controls the notification LED 40. Thereby, the detachment information is notified.

In the medical device system according to the second embodiment, the detachment information is notified by a plurality of devices. Specifically, in the medical device system according to the second embodiment, the detachment information is notified by the muscle relaxation monitoring device 1 (specifically, the notification LED 40), the bedside monitor 20, and the speaker 30.

### Other Embodiments

Hereinafter, other embodiments will be described.

In the embodiments described above, in the comparison determination process of step S2, the calculation process using the first threshold value Ha and the electromyogram waveform is performed, and the process of determining whether the electromyogram waveform has a value larger than the first threshold value Ha continuously for a predetermined time is performed to determine whether the electromyogram waveform exceeds the first threshold value Ha continuously for the predetermined time, but a process of determining whether the electromyogram waveform has a value equal to or larger than the first threshold value Ha continuously for a predetermined time may be performed to determine whether the electromyogram waveform exceeds the first threshold value Ha continuously for the predetermined time.

In the embodiments described above, in the comparison determination process of step S2, the calculation process using the second threshold value Hb and the electromyogram waveform is performed, and the process of determining whether the electromyogram waveform has a value smaller than the second threshold value Hb continuously for a predetermined time is performed to determine whether the electromyogram waveform exceeds the second threshold value Hb continuously for the predetermined time, but the process of determining whether the electromyogram waveform has a value equal to or smaller than the second threshold value Hb continuously for the predetermined time may be performed to determine whether the electromyogram waveform exceeds the second threshold value Hb continuously for the predetermined time.

In the embodiments described above, the counter incremented when it is determined that the value of the electromyogram waveform continuously exceeds the first threshold value Ha for the predetermined time (Y msec) is the same counter as the counter incremented when it is determined that the value of the electromyogram waveform continuously exceeds the second threshold value Hb for the predetermined time (Y msec), but may be a different counter. For example, when it is determined that the value of the electromyogram waveform continuously exceeds the first threshold value Ha for the predetermined time (Y msec), the value of the first counter may be incremented by one, and when it is determined that the value of the electromyogram waveform continuously exceeds the second threshold value Hb for the predetermined time (Y msec), the value of the second counter may be incremented by one. In this case, when the sum of the value of the first counter and the value of the second counter becomes the predetermined value (Z), the detachment information is output to the monitor communication unit 21.

In the embodiments described above, the predetermined time (Y msec) may be shorter than 10 msec. Specifically, the period of the sine wave having the AC frequency of 50 Hz is 1 sec/50 Hz = 20 msec. Since the period of 20 msec is a full wave period, the half wave period is 10 msec, and a value smaller than 10 msec which is the half wave period can be adopted as the predetermined time (Y msec).

In the embodiments described above, the notification LED 40 emits light in the first emission color (for example, green) in the case where the recording electrode 3 is attached, and emits light in the second emission color (for example, red) in the case where the recording electrode 3 is not attached, but the notification LED 40 may not emit light in the case where the recording electrode 3 is not attached, and may emit light in the case where the recording electrode 3 is not attached to notify that the recording electrode 3 is not attached.

In the embodiments described above, the speaker 30 is provided separately from the muscle relaxation monitoring device 1 and the bedside monitor 20, but is not limited thereto. For example, the speaker 30 may be provided in the muscle relaxation monitoring device 1 or may be provided in the bedside monitor 20.

The notification LED 40 is provided in the muscle relaxation monitoring device 1, but is not limited thereto. For example, the notification LED 40 may be provided in the bedside monitor 20 or the speaker 30.

As described above, the muscle relaxation monitoring device 1 according to the presently disclosed subject matter can include the stimulator 8 that stimulates the ulnar nerve of the upper limb L of the patient via the stimulation electrode 2, and the physiological signal detector 9 that detects the physiological signal via the recording electrode 3. Then, the controller 10 detects whether the recording electrode 3 is attached based on the electromyogram waveform corresponding to the physiological signal detected by the physiological signal detector 9. Accordingly, it is possible to provide the muscle relaxation monitoring device 1 capable of detecting whether the recording electrode 3 is attached to the patient without requiring a complicated circuit configuration such as an impedance check function.

Although the embodiments of the presently disclosed subject matter is described with reference to the drawings, specific configurations are not limited to the embodiments, and changes in design and the like without departing from the gist of the presently disclosed subject matter are also included in the presently disclosed subject matter. The embodiments illustrated in the drawings can be combined with each other as long as there is no contradiction or problem in the purpose, configuration, and the like. In addition, the contents described in each drawing can be independent embodiments, and the embodiments of the presently disclosed subject matter are not limited to one embodiment in which each drawing is combined.

In the muscle relaxation monitoring device according to the presently disclosed subject matter, the detachment detector performs a detachment determination process of determining whether the recording electrode is detached every predetermined period.

In the muscle relaxation monitoring device according to the presently disclosed subject matter, in the detachment determination process, it is determined that the recording electrode is detached based on a determination that a calculation result using the physiological waveform and a predetermined threshold value is a specific result.

In the muscle relaxation monitoring device according to the presently disclosed subject matter, the predetermined threshold value includes a first threshold value that is a positive value and a second threshold value that is a negative value, and in the detachment determination process, it is determined that the recording electrode is detached based on a determination that a calculation result using the physiological waveform and the first threshold value or the second threshold value is the specific result.

In the muscle relaxation monitoring device according to the presently disclosed subject matter, in the detachment determination process, it is determined that the recording electrode is detached based on a fact that the specific result continues for a predetermined time.

In the muscle relaxation monitoring device according to the presently disclosed subject matter, in the detachment determination process, when the specific result continues for the predetermined time, a counting process of performing predetermined counting is performed, and it is determined that the recording electrode is detached based on a result of the counting by the counting process.

A medical device system according to the presently disclosed subject matter includes a stimulator configured to stimulate a nerve of a living body via a stimulation electrode attached to the living body, a physiological signal detector configured to detect a physiological signal via a recording electrode attached to the living body, a detachment detector configured to detect detachment of the recording electrode based on a physiological waveform corresponding to the physiological signal detected by the physiological signal detector, a communication unit configured to output detachment information on the detachment of the recording electrode in a case where the detachment of the recording electrode is detected by the detachment detector, and a medical device controller configured to control a medical device based on input of the detachment information output from the communication unit.

### REFERENCE SIGNS LIST

1 muscle relaxation monitoring device
2 stimulation electrode
2a first stimulation electrode
2b second stimulation electrode
3 recording electrode
3a first recording electrode
3b second recording electrode
4 neutral electrode
5 wiring
5a first wiring
5b second wiring
6 signal line
6a first signal line
6b second signal line
7 ground line
8 stimulator
9 physiological signal detector
10 controller
11 operation unit
12 memory
13 communication unit
20 bedside monitor
21 monitor communication unit
22 monitor controller
23 monitor memory
30 speaker
31 audio communication unit
32 audio controller
33 audio memory
40 notification LED
L upper limb
Ha first threshold value
Hb second threshold value

## Claims

1. A muscle relaxation monitoring device comprising:
a stimulator configured to stimulate a nerve of a living body via a stimulation electrode attached to the living body;
a physiological signal detector configured to detect a physiological signal via a recording electrode attached to the living body; and
a detachment detector configured to detect detachment of the recording electrode based on a physiological waveform corresponding to the physiological signal detected by the physiological signal detector.

2. The muscle relaxation monitoring device according to claim 1, wherein
the detachment detector performs a detachment determination process of determining whether the recording electrode is detached every predetermined period.

3. The muscle relaxation monitoring device according to claim 2, wherein
in the detachment determination process, it is determined that the recording electrode is detached based on a determination that a calculation result using the physiological waveform and a predetermined threshold value is a specific result.

4. The muscle relaxation monitoring device according to claim 3, wherein
the predetermined threshold value includes a first threshold value that is a positive value and a second threshold value that is a negative value, and
in the detachment determination process, it is determined that the recording electrode is detached based on a determination that a calculation result using the physiological waveform and the first threshold value or the second threshold value is the specific result.

5. The muscle relaxation monitoring device according to claim 3 or claim 4, wherein
in the detachment determination process, it is determined that the recording electrode is detached based on a fact that the specific result continues for a predetermined time.

6. The muscle relaxation monitoring device according to claim 5, wherein
in the detachment determination process,
when the specific result continues for the predetermined time, a counting process of performing predetermined counting is performed, and
it is determined that the recording electrode is detached based on a result of the counting by the counting process.

7. A medical device system comprising:
a stimulator configured to stimulate a nerve of a living body via a stimulation electrode attached to the living body;
a physiological signal detector configured to detect a physiological signal via a recording electrode attached to the living body;
a detachment detector configured to detect detachment of the recording electrode based on a physiological waveform corresponding to the physiological signal detected by the physiological signal detector;
a communication unit configured to output detachment information on the detachment of the recording electrode in a case where the detachment of the recording electrode is detected by the detachment detector; and
a medical device controller configured to control a medical device based on input of the detachment information output from the communication unit.
